# EUROPEAN PATENT SPECIFICATION

(11) **EP 0 580 305 B1**
(45) Date of publication and mention of the grant of the patent: **22.09.1999**
(21) Application number: 93305184.9
(22) Date of filing: 01.07.1993
(51) Int. Cl.: C07H 1/06, C07H 21/00, C12N 15/10, B01D 71/82, C12N 11/08

(54) **Method and material for purification of nucleic acids**
Verfahren und Material für Reinigung von Nukleinsäuren
Procédé et matériau pour la purification d'acides nucléiques

(30) Priority: 02.07.1992 US 906884
(43) Date of publication of application: 26.01.1994
(73) Proprietor: Edge Biosystems, Inc., Gaithersburg, Maryland 20879 (US)
(72) Inventor: Seed, John, Ellicott City, Maryland 21043 (US); Seed, Brian, Boston, Massachusetts 02114 (US)
(74) Representative: Baker, Colin John

(56) References cited:
- EP-A- 0 268 946
- EP-A- 0 366 438
- EP-A- 0 524 800
- US-A- 4 923 978
- PATENT ABSTRACTS OF JAPAN vol. 13 no. 408 (C-634) ,8 September 1989 & JP-A-01 148186 (TOSOH CORP) 9 June 1989,

## Description

This invention relates to the area of purification and manipulation of nucleic acids.

A variety of different methods for the purification of nucleic acids have been described. Phenol is often used to extract proteins and other material that may interfere with subsequent analysis or manipulation of the DNA. (Rirby, Progr. Nucl. Acid Res. Mol. Biol. vol. 3, p. 1, 1964.) However, phenol poses several safety and health hazards, and is therefore undesirable for occupational safety. Chloroform is also regularly used in the isolation of DNA and RNA, usually in combination with phenol. (Maniatis, et al., Molecular Cloning. A Laboratory Manual, Cold Spring Harbor laboratory, Cold Spring Harbor, N.Y., 1982, pp. 458-460.) Chloroform is a carcinogen, and so, it too, should be eliminated from the workplace.

McCormack U.S. 4,923,978 teaches the use of a solid-phase material with a large surface area and a high concentration of mildly acidic hydroxyls, such as silica, for the separation of proteins from nucleic acids. Svec, U.S. 4,889,632 and 4,923,610, teach the use of a macroporous polymeric membrane which is made from a copolymer of a vinyl monomer (which may be hydroxystyrene among other things) and a divinyl monomer. These are demonstrated as having utility in the separation of proteins. Due to the burgeoning of genetic engineering, there is a continued need for safe, rapid methods for purifying and manipulating nucleic acids.

It is an object of the invention to provide methods for isolating nucleic acids, specifically RNA and DNA.

It is an object of the invention to provide a method for enzymatically manipulating nucleic acids using solid phase materials.

It is still another object of the invention to provide a composition for purifying nucleic acids.

These and other objects of the invention are provided by one or more of the embodiments described below.

In one embodiment of the invention a method is provided for purifying proteins from nucleic acids. A composition is contacted with a substrate under conditions where protein binds and nucleic acids do not. Such conditions are pH 2 to 11 and salt greater than 0.1 mM. The non-binding components are removed from the substrate to yield a substantially protein-free preparation of nucleic acids. The substrate is made by the process of: applying an adhesive formulation to a substrate, said adhesive formulation comprising a non-proteinaceous polymer of monomeric units comprising an aromatic moiety substituted with at least one hydroxyl group with a pK less than 9.

In another embodiment of the invention both proteins and nucleic acids are bound to a substrate and nucleic acids are selectively eluted. The substrate is made by the process of: applying an adhesive formulation to a substrate, said adhesive formulation comprising a non-proteinaceous polymer of monomeric units comprising an aromatic moiety substituted with at least one hydroxyl group with a pK less than 9. The binding step is conducted at pH 2 to 11 and at greater than 0.1 mM salt. The elution is with a buffer having a pH of 4 to 11.

In yet another embodiment of the invention double stranded nucleic acids are purified from both protein and single stranded nucleic acids. When contacted at pH 7 to 10, greater than 1 mM monovalent cation and/or greater than 0.1 mM multivalent cation, both protein and single stranded nucleic acids bind to a substrate made by the process of: applying an adhesive formulation to a substrate, said adhesive formulation comprising a non-proteinaceous polymer of monomeric units comprising an aromatic moiety substituted with at least one hydroxyl group with a pK less than 9. Under these conditions, double stranded nucleic acids do not bind significantly and can be collected in a purified form by simply separating the non-bound components from the substrate.

In still another embodiment of the invention single stranded nucleic acids are separated from double stranded by contacting a composition comprising both to a substrate under conditions of pH 7 to 10, and greater than 1 mM monovalent cation and/or greater than 0.1 mM multivalent cation. The substrate is formed by the process of: applying an adhesive formulation to a substrate, said adhesive formulation comprising a non-proteinaceous polymer of monomeric units comprising an aromatic moiety substituted with at least one hydroxyl group with a pK less than 9. Under such conditions single stranded but not double stranded nucleic acids will bind. Non-binding components can be obtained by simply separating them from the substrate.

In still another embodiment of the invention an enzyme is bound to a substrate made by the process of: applying an adhesive formulation to a substrate, said adhesive formulation comprising a non-proteinaceous polymer of monomeric units comprising an aromatic moiety substituted with at least one hydroxyl group with a pK less than 9. The enzyme uses nucleic acids as a substrate. Nucleic acids are added to the substrate bound enzyme under conditions where the nucleic acids do not bind and the enzyme is catalytically active. After the reaction has achieved the desired degree of completion, nucleic acids can be separated from the substrate by simple phase separation. The enzyme is bound to the substrate under conditions which minimize changes in activity. The range of possible conditions include pH 2 to 11, and greater than 0.1 mM salt. The enzyme reaction occurs at appropriate conditions for catalytic activity within the range of pH 4 to 11.

In yet another embodiment of the invention a composition is provided which comprises a substrate coated with an adhesive formulation comprising a nonproteinaceous polymer of monomeric units comprising an aromatic moiety substituted with at least on hydroxyl group with a pK less than 9.

These and other embodiments, which will be described in more detail below, provide the art with rapid and safe means of purifying and manipulating nucleic acids.

It has now been found that nonproteinaceous polymers, in a solid or semi-solid state, specifically bind to proteins. Such polymers bear a favorable proportion of aromatic monomeric units which are substituted on the aromatic moiety with at least one hydroxyl group. The pK of the hydroxyl group is less than 9. The discovery of such a solid or semi-solid for protein binding has application with regard to purification of nucleic acids and enzymatic modifications of nucleic acids.

Aromatic monomeric units according to the present invention are chemical structures which have a resonance stabilized, conjugated ring system. These include hydrocarbons, organic or inorganic heterocycles, and elementally pure inorganic compounds. The resonance stabilized, conjugated ring structure is preferably planar. As mentioned above, the ring structure carries at least one hydroxyl group with a pK less than 9. Other substitutions on the ring system or on the polymer backbone may be used. Some may modulate the affinity of the polymer for proteins. Some may also permit the cross-linking of the polymer. Others may be used to irreversibly chemically bond to proteins.

The polymer may be a random copolymer, a block copolymer, or a graft copolymer. The aromatic moiety is preferably a phenyl group. Suitable aromatic monomeric units for polymer formation include hydroxyphenyl ethyl-containing groups, especially para-hydroxyphenyl ethyl groups. The ring or polymer backbone may contain other substituents. The polymer is nonproteinaceous, i.e., it is not a naturally occurring, semi-synthetic, or synthetic protein. The backbone of the polymer is not made up of peptide bonds. Typically the aromatic monomeric units will not be amino acids.

Various substituents, proportions of aromatic monomeric units, and resonance-stabilized, conjugated ring systems can be readily tested for use in the present invention by contacting formulations containing them with a surface, removing excess formulation, if necessary, contacting the treated surface with a solution, suspension, powder or aerosol of protein, removing excess protein, if necessary, and measuring the amount of protein bound to the surface or removed from the solution, suspension, etc. Such measurement may be by dye binding, spectroscopy, scintillation counting in the case of radiolabeled protein, enzymatic activity, antibody binding, or direct measurement of the capacity of the treated surface to perform in the desired application.

The polymer can be formulated as an adhesive or can be used as a solid, such as in the form of beads. Preferably the adhesive will be applied as a coating to a substrate to form a device for purification of nucleic acids. An adhesive formulation according to the present invention contains as its active ingredient the aromatic hydroxylated polymer described above. It may also have one or more diluents, thickening agents, dispersing agents, dyes, or surface conditioning agents. Typically the adhesive formulation will be prepared in an aqueous solution or in a polar organic solvent. Suitable solvents include ethanol, ethyleneglycol diacetate, ethyl acetate, acetone and dimethylsulfoxide. Beads may be impregnated with fluorescent or absorptive dyes, or magnetic or paramagnetic particles.

Proteins or protein-containing compositions according to the present invention include purified and crude preparations of protein, such as cell lysates. Nucleic acids according to the present invention include purified as well as crude preparations such as cell lysates, and also include single-stranded as well as double-stranded, and synthetic as well as natural polymers.

Substrates which may be coated with the adhesive formulation of the present invention include plastics, natural or synthetic fibers or fabrics, membranes or papers, metal, glass, and ceramic. Suitable plastics include substituted polyalkene, polyamide, polyester, polyether, polysulfone, phenolic resin, epoxy resin, and substituted cellulose. These can be coated or partially coated by any means known in the art, including spraying, dipping, brushing, stamping, extruding, or perfusing. The coated substrates of the present invention may take many forms. These include beads, strips, wells in multi-well plates, strips or individual units, tubing, mesh, open-cell foam, membranes, papers, needles, fibers, plates, slides and cell culture vessels.

Proteins bind to the coated substrates of the present invention under a broad range of conditions. As the pH increases, the binding decreases, generally. Conditions of pH 2 to 11 may be used, with pH 4 to 10 forming the preferred range. At constant pH, as the cation concentration increases, so does protein binding. Cation concentrations greater than 0.1 mM for multivalent cations and 1 mM for monovalent cations may be used. The minimum cation concentration necessary to produce an effect varies as a function of pH and buffer. Generally, the lower the pH, the lower the minimum cation concentration necessary to promote binding. Thus, the cation concentration necessary to produce a given amount of protein binding will be significantly greater at pH 10 than at pH 7. Examples of useful inorganic, multivalent cations include magnesium, copper, and lanthanum. Examples of useful inorganic, monovalent cations include sodium, potassium and lithium. Cation concentrations greater than 1 M do not substantially increase protein binding within the pH range of 4 - 10. (Doubling the cation concentration to 2 M generally increases protein binding by less than 15%.) Multivalent cations are more efficient than monovalent cations to promote binding, with chloride being a more efficient counterion than acetate and trichloroacetate to promote binding. Chaotropic organic cations tend not to be effective at promoting protein binding. Other organic cations, those bearing primary amines in particular, generally are less effective than inorganic cations. The increase in protein binding associated with a decrease in pH is generally independent of the cation effect. Thus, the maximum amount of protein binding varies as a function of pH. The same is true of nucleic acid binding to the substrate.

Double stranded DNA binds to the coated substrate better as the pH decreases. Conditions of pH 2 to 11 may be used, with pH 4 to 10 forming the preferred range. At constant pH, as the cation concentration increases, so does nucleic acid binding, generally. Binding of double stranded nucleic acids in the presence of cations tends to be dependent on the valence state and concentration of the cation. Higher valence states promote binding more effectively than lower valence states. The relative effectiveness of different cation valence states is +3>+2>+1. In particular, La⁺³> > Ca⁺²> Mg⁺² ≈ Cu⁺² > K⁺ ≈ Na⁺. Concentrations of greater than 0.01 M monovalent cation may be used, with concentrations greater than 0.1 M being preferred. In contrast to the higher valence state cations, the pH range for stimulation of binding by monovalent cations is limited to 2 - 7, with a preferred range of 4 - 7. As with protein binding, concentrations greater than 1 M do not substantially increase the amount of DNA bound to the substrate. (Doubling the cation concentration to 2 M generally increases nucleic acid binding by less than 15 %.) Divalent cations increase binding more effectively than monovalent cations. These include, among others, transition metal cations such as copper, and alkaline earth cations such as calcium and magnesium. For divalent cations, concentrations greater than 1 mM may be used, with concentrations greater than 10 mM being preferred. For cations such as lanthanum, with higher valency states, concentrations greater than 0.1 mM may be used. Multivalent cation concentrations greater than 1 M do not substantially increase DNA binding. However, the small increases in DNA binding at concentrations greater than 1 M may be important in some cases to achieve binding efficiencies greater than 90%.

Single stranded nucleic acids also bind to the coated substrate of the invention. Binding increases as the pH decreases, generally. DNA oligonucleotides tend to bind better to the coated substrate of the invention than RNA at any given pH and cation concentration in the range of pH 7 - 10. Single stranded nucleic acids are more sensitive to the effects of pH and monovalent or divalent cations than double stranded DNA. However, for cations with valency states greater than 2, such as lanthanum, binding of double and single stranded nucleic acids to the substrate of the invention is similar. Monovalent cations increase single strand binding at concentrations greater than 1 mM. Multivalent cations also promote binding, and generally do it more effectively. Concentrations greater than 0.1 mM can be used. The minimum cation concentration necessary to produce an effect varies as a function of pH and buffer. Generally, the lower the pH the lower the minimum concentration of cation necessary to promote binding. Chloride counterions promote binding more effectively than acetate.

Separation of double stranded nucleic acids from single stranded nucleic acids is possible because of marked quantitative differences in binding of the nucleic acids to coated substrates of the invention as a function of cation concentration. The mildly basic pH range (7 - 10) is optimal for separation of double stranded and single stranded nucleic acids. In this pH range, monovalent cations have little or no effect on DNA binding even at concentrations of 1 M or better whereas binding of single stranded nucleic acids is stimulated under these conditions. Divalent cation concentrations required to promote double strand DNA binding in this pH range are up to 100 times greater than those required to promote a similar level of single stranded nucleic acid binding. Magnesium salts are particularly useful in this regard. Some separation of single and double stranded nucleic acids occurs in the acidic pH range due to the lower sensitivity of the double stranded DNA to the effects of salt on nucleic acid binding. However, this separation is generally more difficult to effect because of increased binding of double stranded DNA especially if the DNA contains single base overhangs such as those produced by restriction with Hind III.

Nucleic acids can be eluted from the coated substrate by shifting pH and/or cation concentrations to those which are less favorable to binding, i.e., by increasing pH and/or reducing cation concentration. Generally, the greater the difference between binding conditions and elution conditions, the more efficient the elution of the nucleic acid. The preferred condition for elution of a nucleic acid is at a pH and cation concentration where no binding of said nucleic acid can occur. For example, DNA oligonucleotides, bound to a coated substrate at pH 7.5 in the presence of 10 mM MgCl₂ can be eluted from the substrate at the same pH in the presence of 5 mM MgCl₂. If the pH is increased to 9 and the MgCl₂ concentration dropped to 1 mM, then the nucleic acid elutes more efficiently. This increase in efficiency generally is beneficial since it results in a substantially smaller elution volume. If a multivalent cation is used to bind the nucleic acid to the substrate, a chelator can be added, such as ethylenediaminetetraacetate (EDTA). Generally chelators are used in an amount sufficient to chelate the multivalent cations used in binding the DNA to the substrate. However, EDTA, in particular, also will enhance dissociation of nucleic acids from the substrate even when monovalent cations are used to bind the nucleic acid to the substrate. This effect may relate to the deleterious action of acetate counterions on nucleic acid and protein binding. Ranges of pH from 4 to 11 can be used to elute double or single stranded nucleic acids, with a preferred pH range of 6 -10. Generally, the pH range of 4 - 8 can be used most effectively for the elution of double stranded nucleic acids, since single stranded nucleic acid binding to the substrate is apparent at pH 4 - 8 even in the absence of multivalent cations and in the presence of low (0.01 M) monovalent cation concentrations. Nevertheless, elution of single stranded nucleic acids is possible in this pH range, albeit inefficient. For double stranded nucleic acids, monovalent cation concentrations less than 1 M and divalent cation concentrations less than 0.1 M, may be used with a preferred concentration of less than 0.1 M for monovalent and 0.01 M for divalent cations. A pH range of 4 - 11 may be used, with 6 - 10 forming the preferred range. For single stranded nucleic acids, concentrations less than 0.5 M monovalent cation and less than 10 mM divalent cation may be used, with preferred concentrations of less than .05 M for monovalent cations and 1 mM for divalent cations. Acetate counterions are preferred over chloride. Above pH 7, salt concentrations as high as 1 M may be used, although salt concentrations less than 0.1 M are preferred. For single stranded nucleic acids, the pH range for elution is 7 - 11, with pH 8 - 10 forming the preferred range.

According to one method of the present invention, a nucleic acid- and protein-containing composition is contacted with the coated substrate of the invention. The protein or nucleic acid may adhere by non-covalent forces, or alternatively may be cross-linked to the adhesive formulation by covalent interactions. As described previously, interaction of protein and nucleic acid with the substrate is affected by pH and salt. A decrease in pH or increase in ionic strength results in increased protein and nucleic acid adherence. The relative sensitivity of protein and nucleic acids to this effect is protein>single stranded nucleic acid>double stranded nucleic acid. When both protein and DNA are present, protein competes for binding sites more effectively than DNA. Under conditions where the adherence is by non-covalent forces, the protein and nucleic acids can be dissociated from the substrate by contacting the substrate with a composition with increased pH and decreased salt. The relative sensitivity is double stranded nucleic acid>single stranded nucleic acid > > protein. As described previously, cations will enhance binding of proteins and nucleic acids to the substrate of the invention. Therefore, under most conditions, the lower the salt concentration, the more effective the elution of the bound nucleic acid. However, at very low ionic strength, protein may also dissociate from the substrate. Thus, the choice of salt concentration for dissociation of nucleic acids from substrate containing both protein and nucleic acid, falls within the same ranges as described previously for dissociation of nucleic acid from the substrate, but will be influenced by the need to retain bound protein while eluting the desired material. Dissociation of protein from the substrate is sufficiently slow that under most conditions, little or no detectable protein is lost from the substrate when the pH is raised and salt decreased, even though the amount of protein adhering to the substrate under the dissociation conditions is greater than the maximum amount which can be transferred from solution to substrate under the same conditions. Nevertheless, significant amounts of protein may dissociate from the substrate if the initial binding conditions are at or near saturation.

When multivalent cations are used to adhere protein and nucleic acid, the adherent materials also can be dissociated from the substrate by addition of metal ion chelating agents, such as EDTA. The relative sensitivity to these agents is double stranded nucleic acid>single stranded nucleic acid > > protein. Release of bound protein is more likely to occur in solutions containing chelating agent such as EDTA than in solutions where only the pH is adjusted. The net effect of these pH and salt dependent differences is that double stranded nucleic acids (primarily DNA) can be separated from single stranded nucleic acids and/or protein at mildly basic pH, by binding the single stranded material and protein to the substrate and allowing the double stranded material to pass through. In addition, single stranded nucleic acids can be separated from double stranded nucleic acids by following the above approach, then eluting the single stranded nucleic acid from the substrate by increasing pH and reducing salt and/or adding chelating agent. The useful ranges of cation concentration and/or pH have been described previously. The choice of pH, cation, and cation concentration for a particular protocol is regulated by a variety of parameters, including extent and selectivity of nucleic acid binding, usefulness or possible interference of the salt or pH in subsequent manipulations of the eluted material, the amount of protein and/or nucleic acid which must be bound or eluted and the composition of the nucleic acid (e.g., length, single or double stranded, RNA or DNA).

There are many specific uses to which the adhesive-coated substrates and polymeric beads of the present invention can be put. As described above, they can be used to remove proteins from nucleic acids in a sample, such as a cell lysate or a restriction enzyme digest. They can be used to separate and isolate single stranded and double stranded nucleic acids from complex compositions by using conditions which selectively enhance the affinity of one nucleic acid for the substrate or by using conditions which selectively enhance dissociation of a nucleic acid from the substrate. An example of this would be purification of a DNA amplification product from the polymerase chain reaction.

The adhesive coated substances can also be used to perform solid phase enzymatic reactions in which enzymatically active protein is bound to substrate and the nucleic acid is in solution in a composition in contact with the substrate. They can be used to alter the extent of enzymatic reactions in which the reactant is a double stranded nucleic acid and the product is a single stranded nucleic acid. These features can be used in a variety of combinations to provide systems for rapid processing and manipulation of nucleic acids for purposes such as sequencing.

As mentioned above, based on the differential binding of protein, double stranded, and single stranded nucleic acids, a variety of practical applications are feasible. In one such application, the coated substrate is used to purify the amplified double stranded product of polymerase chain reaction from the proteins and primers necessary for the reaction. If the reaction mixture is contacted with a coated substrate of the invention at pH 7.0 to 10 and greater than 1 mM multivalent cation, then proteins and primers bind, while double stranded product does not. Two particularly suitable buffers for carrying out this purification (because of their utility in subsequent manipulations such as sequencing) are: 0.02 M Tris-HCl, 0.05 M KCl or NaCl, 10 mM MgCl₂, pH 7.5, and 0.01 M Tris-HCl, 0.05 M KCl, 15 mM MgCl₂, pH 8.3.

Another practical application of the coated substrate is for purification of plasmid DNA from bacterial cells. Briefly, a plasmid-containing bacterial pellet is resuspended in 10 mM Tris-HCl, 1 mM EDTA, 5% glycerol, pH 8.0 (TE) or preferably 5% glycerol, with or without RNase A. A lysis buffer (0.2-0.4 N NaOH, 0.5-1% SDS) is added. After at least 10 seconds, a neutralization buffer is added (1-2 MKCl, 0.1-0.5 M HCl, 0.05-0.5 M buffer [pK 4-10], 0-0.2 M multivalent salt. The mixture is mixed by vortex and loaded onto a cartridge comprising a coated substrate. (See example 10, infra, for description of the cartridge.) After the sample wicks into the matrix, a solution containing 1 M KCl and carriers is added. The eluate can be centrifuged into a receiver tube and then 2 volumes of ethanol added to precipitate the DNA. The precipitate is centrifuged for 2 minutes at greater than 10,000 x g for 2 minutes to pellet DNA. Alternatively, the eluate can be centrifuged directly into 2 volumes of ethanol at >10,000 x g for 2 minutes to pellet DNA. The pelleted DNA can be rinsed with 70% ethanol, dried and dissolved in TE.

### EXAMPLES

### Example 1.

This example demonstrates the purification of restriction enzyme digested DNA from the restriction enzyme. The enzyme bound to a coated membrane substrate of the invention while DNA passed through.

3.3µg of Hind III restricted λ DNA (0.1 A₂₆₀ units in 0.6 ml) was added to 50 µl of buffer containing 50 mM Tris-HCl, pH 7.5 (at 37°C), 100 mM NaCl, 10 mM MgCl₂, 1 mM mercaptoethanol and 50 U Hind III. 2 µl of 0.5 M EDTA was added to the buffer and solution was placed in a DNA purification cartridge. The cartridge was open on one end and physically restricted at the other by a polypropylene grid which supported a porous polyethylene disk (15-30 µ nominal pore size) coated with poly-4-hydroxystyrene (PHS), which in turn supported a PHS-coated nitrocellulose membrane (0.1 µ pore size before coating). The coating process consisted of immersing the membrane or porous polyethylene in a solution of PHS (100,000 MW) in 50% aqueous ethanol at 50 mg/ml, removing excess polymer and drying the membrane or porous plastic at room temperature in a stream of filtered air. The disk and membrane were held in place in the cartridge with a teflon sleeve which prevented leakage of the sample around the edges of the membrane. The cartridge was placed in and supported by a 1.5 ml microcentrifuge tube which served as the receiver tube for purified DNA. The solution containing DNA and enzyme was dispersed evenly over the membrane in the restricted end of the cartridge. A cap which was attached to and which fit tightly over the open end of the cartridge was pushed into place. The action of pushing the cap into place created positive pressure within the cartridge, thereby forcing the DNA-containing solution through the membrane, porous polyethylene frit and polypropylene grid and into the 1.5 ml microcentrifuge receiver tube. The tube and cartridge were centrifuged together at full speed on a microcentrifuge for 5 seconds to insure maximum recovery of fluid volume. Fluid recovery was 47 µl. To recover the retained material (5 µl), 10 µl of distilled, deionized water was added to the cartridge, dispersed over the membrane, the lid of the cartridge closed and the cartridge and receiver tube centrifuged for 5 seconds in a microcentrifuge. The amount of DNA recovered in the eluate was 97% of the amount applied (determined spectrophotometrically at 260 nm). No residual restriction endonuclease activity could be detected in an overnight (16 hr) incubation of the cartridge eluate with pUC19 DNA. Passage of endonuclease buffer through the cartridge does not significantly alter its composition or inhibit the activity of added restriction endonuclease.

### Example 2.

This example demonstrates the high binding capacity of a coated substrate of the invention.

BSA was applied to the cartridge described in example 1 and extracted as described in example 1, in buffer containing 50 Mm Tris-HCl, pH 7.5 (at 37°C), 10 mM MgCl₂, 100 mM NaCl and 1 mM dithioerythritol, in a volume of 50 µl, in amounts of 10, 20, 30 and 40 µg. The amount of protein detected in the eluate was 0 µg for 10, 20 and 30 µg loads and 0.1 µg for a 40 µg load.

### Example 3.

This example demonstrates the pH dependence of single stranded RNA binding to a coated substrate of the invention.

RNA (3.2 µg) from MS2 bacteriophage was applied to the cartridge described in example 1, and processed as described in example 1, using 100 µl of buffer comprising 1 M KCl and 0.1 M Tris-HCl (varied from pH 7.0 to 8.5 in pH increments of 0.5). The amount of RNA recovered in the eluate was determined spectrophotometrically at 260 nm. RNA recovery was 24% at pH 7.0, 42% at pH 7.5, 60% at pH 8.0 and 85% at pH 8.5.

### Example 4.

This example demonstrates the pH dependence of double stranded DNA binding to a coated substrate of the invention. Above pH 7.0 essentially no DNA binds.

DNA (3.3 µg) from φX174, restricted with Hae III was applied to the cartridge described in example 1, and processed as described in example 1. The pH of the buffer was varied from 7.0 to 8.5 in increments of 0.5 units. DNA recovery was 96% at pH 7.0, 100% at pH 7.5, 100% at pH 8.0 and 96% at pH 8.5.

### Example 5.

This example demonstrates the salt concentration dependence at pH 7.5 of a single stranded oligomer's binding to a coated substrate of the invention.

2 µg of a 20 nucleotide oligomer (0.100 A₂₆₀ units in 0.6 ml), sequence 5'-GGAAACAGCTATGACCATGA-3' was processed through the cartridge as described in example 1, using buffer comprising 0.01 M Tris-HCl, pH 7.5, 0.05 M KCl and MgCl₂ concentrations of 1, 3, 5, and 10 mM. The recovery of oligomer was 35% at 1 mM MgCl₂, 11% at 3 mM MgCl₂, 3% at 5 mM MgCl₂, and 0% at 10 mM MgCl₂.

### Example 6.

This example demonstrates the salt concentration dependence at pH 7.5 of double stranded DNA to a coated substrate of the invention.

3.3 µg of DNA from φX174 was processed as described in example 5. The recovery of DNA was 91% at 1 mM MgCl₂, 91% at 3 mM MgCl₂, 90% at 5 mM MgCl₂, and 87% at 10 mM MgCl₂.

### Example 7.

This example demonstrates the sequential binding and elution of double stranded DNA to a coated substrate of the invention. At pH 4.0, 1.9 M MgCl₂, 90% of the DNA bound. At pH 8.5, 20 mM Tris-HCl, 20 mM EDTA, about 80% eluted.

3.3 µg of DNA φX174 was applied to the cartridge described in example 1 and processed as described in example 1, using a buffer comprising 0.02 M sodium acetate pH 4.8 and 1.9 M MgCl₂. The amount of DNA in the eluate was 10%. 100 µl of H₂O was added to the cartridge, the lid shut and the cartridge centrifuged briefly to recover the water wash. The DNA was then eluted by adding a solution comprising 0.02 M Tris-HCl, 0.02 M EDTA, pH 8.5. The lid was snapped shut and the eluate collected following a brief 5 second centrifugation. The amount of DNA recovered in the eluate was 79% of the amount applied.

### Example 8.

This example demonstrates that at conditions where most DNA elutes (pH 8.5, 20 mM Tris-HCl, 20 mM EDTA) essentially all protein remains bound to the coated substrate of the invention.

20 µg of BSA was processed through a cartridge as described in example 7. No protein was detected in either the sample eluate, water wash or the pH 8.5 elution.

### Example 9.

This example demonstrates that RNA can be recovered in good quality from crude lysates of human lymphocytes in the absence of protein.

5 x 10⁶ human lymphocytes isolated from a healthy male volunteer were lysed with 300 µl of 0.5% SDS buffered with 0.01 M Tris-HCl, pH 8.5, containing 100 µg/ml proteinase K and 10 mM vanadyl ribonucleoside complex. An equal volume of 2 M KCl was immediately added and vortexed. The resulting suspension was loaded into a cartridge similar to that described in example 1 with the following alterations. The PHS-coated porous polyethylene frit and nitrocellulose membrane were placed in the tip end of a 3 cc syringe barrel. The frit and membrane were held in place by the outside rim of a cartridge containing 1) a polypropylene grid in the base (which was in contact with the membrane) and 2) a 3 cm cylinder of axially-oriented, PHS-coated polyester fibers (coated as described in example 1) which were compressed to a length of 1.5 cm. The syringe barrel cartridge was centrifuged in a Sorvall GLC centrifuge at 3,000 rpm for 2 minutes. The eluate was precipitated with ethanol and the product electrophoresed on an agarose gel. The resulting preparation of RNA was of good quality as evidenced by the ribosomal RNA bands on the gel, and the preparation contained no detectable protein.

### Example 10.

This example demonstrates the use of coated substrates to purify plasmid DNA from rapid lysates.

A culture of *E. coli* carrying the ampicillin resistance plasmid pUC19 was grown to saturation overnight at 37°C in Luria Broth with vigorous agitation. 1.5 ml of bacterial suspension was pelleted by centrifugation at 13,000 x g in a microcentrifuge for 20 seconds. The clear supernatant was aspirated and the pellet resuspended by vigorous mixing in 60 µl of 10 mM Tris-HCl, 1 mM EDTA, pH 8.0, containing 1 µg of RNase A. The cell suspension was lysed by the addition of 60 µl of 0.3 N NaOH containing 1% sodium dodecyl sulfate. 60 µl of a solution containing 1.9 M KCl, 0.1 M Tris and 0.36 M HCl was added to the lysed cell suspension and vortexed vigorously. The resulting suspension was transferred to a cartridge similar to that described in example 1, except that the cartridge also contained a cylindrical bundle of PHS-coated, axially oriented, polyester fibers cut to a length of 1.3 cm. After the sample wicked into the fiber bundle, it was chased with 100 µl of 1 M KCl containing carriers for DNA precipitation. The cartridge was placed in a 2 ml microcentrifuge receiver tube containing 0.5 ml ethanol and centrifuged at full speed (13,000 x g) for 2 minutes. The resulting pellet in the 2 ml microcentrifuge tube was washed with 70% ethanol, dissolved in TE, and electrophoresed on a 1% agarose gel. The results showed better recovery of plasmid DNA compared to standard (Birnboim and Doly, *Nucl*. *Acids Res*. Z, 1513-23, 1979) alkaline lysis minipreps, without detectable quantities of protein.

### EXAMPLE 11

This example provides an alternative procedure for preparing plasmid DNA.

A culture of *E. coli* carrying the ampicillin resistance plasmid pUC19 was grown to saturation overnight at 37°C in Luria Broth with vigorous agitation. 1.5 ml of bacterial suspension was pelleted by centrifugation at 13,000 x g in a microcentrifuge for 20 seconds. The clear supernatant was aspirated and the pellet resuspended by vigorous mixing in 60 µl H₂O. The cell suspension was lysed by the addition of 60 µl of 0.3 N NaOH containing 1% sodium dodecyl sulfate. 60 µl of a solution containing 1.5 M KCl, 0.1 M MOPS, 0.05 M MgCl₂, and 0.28 M HCl was added to the lysed cell suspension and vortexed vigorously. The resulting suspension was transferred to a cartridge similar to that described in Example 1, except that the cartridge also contained a cylindrical bundle of PHS-coated, axially oriented, polyester fibers cut to a length of 1.3 cm. After the sample wicked into the fiber bundle, it was chased with 120 µl of 1 M KCl. The cartridge was placed in a 2 ml microcentrifuge receiver tube containing 0.5 ml ethanol and centrifuged at full speed (13,000 x g) for 2 minutes. The resulting pellet in the 2 ml microcentrifuge tube was washed with 70% ethanol and dissolved in TE.

## Claims

1. A method for purifying nucleic acids from proteins, comprising the steps of:
contacting a liquid phase composition comprising protein and nucleic acid with a substrate formed by the process of:
applying an adhesive formulation to a substrate, said adhesive formulation comprising a non-proteinaceous polymer of monomeric units comprising an aromatic moiety substituted with at least one hydroxyl group with a pK less than 9;
said contacting occurring at a pH of 2 to 11 and a cation concentration of greater than 0.1 mM, so that protein binds to said substrates but nucleic acids do not bind to said substrate;
separating the liquid phase from the substrate.

2. A method for purifying nucleic acids from proteins comprising the steps of:
contacting a liquid phase composition comprising protein and nucleic acids with a substrate formed by the process of:
applying an adhesive formulation to a substrate, said adhesive formulation comprising a non-proteinaceous polymer of monomeric units comprising an aromatic moiety substituted with at least one hydroxyl group with a pK less than 9;
said contacting occurring at a pH of 2 to 11 and a cation concentration of greater than 0.1 mM, so that protein and nucleic acids bind to said substrate;
selectively eluting the nucleic acids from the substrate with a buffer having a pH of 4 to 11.

3. A method of purifying double stranded nucleic acids from both proteins and single-stranded nucleic acids, comprising the steps of:
contacting a liquid phase comprising protein and double and single stranded nucleic acids with a substrate formed by the process of:
applying an adhesive formulation to a substrate, said adhesive formulation comprising a non-proteinaceous polymer of monomeric units comprising an aromatic moiety substituted with at least one hydroxyl group with a pK less than 9;
said contacting occurring at a pH of 7 to 10 with one or both of a monovalent cation at > 1mM and a multivalent cation at >0.1 mM being present, so that protein and single stranded nucleic acids bind to said substrate but double stranded nucleic acids do not bind to said substrate;
separating the liquid phase from the substrate.

4. A method for separating single stranded from double stranded nucleic acids comprising the steps of:
contacting a liquid phase comprising double and single stranded nucleic acids with a substrate formed by the process of:
applying an adhesive formulation to a substrate, said adhesive formulation comprising a non-proteinaceous polymer of monomeric units comprising an aromatic moiety substituted with at least one hydroxyl group with a pK less than 9;
said contacting occurring at a pH of 7.0 to 10 with one or both of a monovalent cation at >1 mM and a multivalent cation at >0.1 mM being present, so that single stranded but not double stranded nucleic acids bind to said substrate;
separating the liquid phase from the substrate.

5. A method according to claim 1 wherein said liquid phase has a pH of 4 to 10.

6. A method according to claim 1 wherein said contacting occurs at a pH of 7.5 to 11 and a cation concentration of 0.01 to 1 M.

7. A method according to claim 2 wherein said liquid phase has a pH of 6 to 10.

8. A method according to claim 2 wherein the liquid phase comprises multivalent cations and said buffer for eluting contains sufficient chelating agent to bind all of said multivalent cations.

9. A method according to claim 2 wherein said step of contacting occurs at a pH of 2 to 7 and a cation concentration of 10 mM to 1 M.

10. A method according to claim 2 wherein the eluted nucleic acids are double stranded and said step of selectively eluting occurs at a pH of 6 to 10, and at less than 0.1 M monovalent cation, and at less than 0.01 M multivalent cation.

11. A method according to claim 2 wherein the eluted nucleic acids are single stranded and said step of selectively eluting occurs at a pH of 8 to 10 and at less than 0.5 M cation.

12. A method according to claim 3 wherein said liquid phase is selected from 10 mM MgCl₂, pH 7.5; and 15 mM MgCl₂, pH 8.3.

13. A method according to claim 3 wherein said contacting occurs at a pH less than 8.5 and at greater than 0.5 M cation.

14. A method according to claim 4 wherein said step of contacting occurs at a pH of 8 to 10 and at less than 0.1 M monovalent cation, and at less than 0.01 M multivalent cation.

15. A method for performing an enzymatic reaction to modify nucleic acids, comprising the steps of:
contacting an enzyme which modifies nucleic acids with a solid substrate made by the process of:
applying an adhesive formulation to a solid substrate, said adhesive formulation comprising a non-proteinaceous polymer of monomeric units comprising an aromatic moiety substituted with a least one hydroxyl group with a pK less than 9;
said contacting occurring at a pH of 2 to 11 and a cation concentration of greater than 0.1 mM, so that said enzyme binds to said solid substrate;
contacting said solid substrate-bound enzyme with nucleic acids under conditions appropriate for said enzyme to be catalytically active, said conditions falling within the range of pH to 4 to 11 and greater than 0.1 mM cation;
separating the nucleic acids from the solid substrate to yield enzymatically modified nucleic acids.

16. A method according to claim 15 wherein the first step of contacting occurs between pH 4 and 10.

17. A device for purifying nucleic acids, which comprises:
a substrate coated with an adhesive formulation comprising a nonproteinaceous polymer of monomeric units comprising an aromatic moiety substituted with at least one hydroxyl group with a pK less than 9.

18. A device according to claim 17 wherein the aromatic moiety is a phenyl group.

19. A device according to claim 17 or claim 18 wherein the hydroxyl group is a para-hydroxyl relative to said aromatic moiety's attachment site to said polymer's backbone.

20. A device according to any of claims 17 to 19 wherein the substrate is a membrane.

21. A device according to any of claims 17 to 19 wherein the substrate comprises a plastic.

22. A device according to claim 21 wherein the plastic is selected from substituted polyalkene, polyamide. polyester, polyether, polysulfone, phenolic resin, epoxy resin, and substituted cellulose.

23. A device according to claim 21 or claim 22 wherein the plastic is in the form of beads.

## Patentansprüche

1. Verfahren zur Reinigung von Nukleinsäuren von Proteinen mit den Schritten:
- Kontaktierung einer Flüssigphasenzusammensetzung enthaltend Protein und Nukleinsäure mit einem Substrat, das gebildet wurde durch das Verfahren der Anwendung einer adhäsiven Formulierung auf ein Substrat, wobei die adhäsive Formulierung ein nicht-proteinartiges Polymer aus monomeren Einheiten enthält, die einen aromatischen Kern aufweisen, der mit wenigstens einer Hydroxylgruppe mit einem pK-Wert von weniger als 9 substituiert ist;
- Vornahme der Kontaktierung bei einem pH-Wert von 2 bis 11 und einer Kationenkonzentration von größer als 0.1 mM, so daß Protein an das Substrat bindet aber Nukleinsäuren nicht an das Substrat binden;
- Separierung der Flüssigphase von dem Substrat.

2. Verfahren zur Reinigung von Nukleinsäuren von Proteinen mit den Schritten:
- Kontaktierung einer Flüssigphasenzusammensetzung enthaltend Protein und Nukleinsäuren mit einem Substrat, das gebildet wurde durch das verfahren der Anwendung einer adhäsiven Formulierung auf ein Substrat, wobei die adhäsive Formulierung ein nicht-proteinartiges Polymer aus monomeren Einheiten enthält, die einen aromatischen Kern aufweisen, der mit wenigstens einer Hydroxylgruppe mit einem pK-Wert von weniger als 9 substituiert ist;
- Vornahme der Kontaktierung bei einem pH-Wert von 2 bis 11 und einer Kationenkonzentration von größer als 0.1 mM, so daß Protein und Nuklearsäuren an das Substrat binden;
- Selektive Eluierung der Nukleinsäuren von dem Substrat mit einem Puffer mit einem pH-Wert von 4 bis 11.

3. Verfahren zur Reinigung doppelsträngiger Nukleinsäuren von sowohl Proteinen als auch einsträngigen Nukleinsäuren mit den Schritten:
- Kontaktierung einer Flüssigphase enthaltend Protein und doppel- und einsträngigen Nukleinsäuren mit einem Substrat gebildet durch das verfahren der Anwendung einer adhäsiven Formulierung auf ein Substrat, wobei die adhäsive Formulierung ein nicht-proteinhaltiges Polymer aus monomeren Einheiten aufweist, die einen aromatischen Kern aufweisen, der mit wenigstens einer Hydroxylgruppe mit einem pK-Wert von weniger als 9 substituiert ist;
- Vornahme der Kontaktierung bei einem pH-Wert von 7 bis 10, wobei eines oder beide von einem monovalenten Kation mit > 1mM und ein multivalentes Kation mit >0.1 mM anwesend sind, so daß das Protein und einsträngige Nukleinsäuren an dem Substrat binden aber doppelsträngige Nukleinsäuren nicht an das Substrat binden;
- Separierung der Flüssigphase von dem Substrat.

4. Verfahren zur Separierung einsträngiger von doppelsträngigen Nukleinsäuren mit den Schritten:
- Kontaktierung einer Flüssigphase mit doppel- und einsträngigen Nukleinsäuren mit einem Substrat gebildet durch das verfahren der Anwendung einer adhäsiven Formulierung auf ein Substrat, wobei die adhäsive Formulierung ein nicht-proteinhaltiges Polymer aus monomeren Einheiten aufweist, die einen aromatischen Kern aufweisen, der mit wenigstens einer Hydroxylgruppe mit einem pK-Wert von weniger als 9 substituiert ist;
- Vornahme der Kontaktierung bei einem pH-Wert von 7 bis 10, wobei eines oder beide von einem monovalenten Kation mit > 1mM und ein multivalentes Kation mit >0.1 mM anwesend sind, so daß einsträngige aber nicht doppelsträngige Nukleinsäuren zu dem Substrat binden aber nicht doppelsträngige Nukleinsäuren an das Substrat binden;
- Separierung der Flüssigphase von dem Substrat.

5. Verfahren nach Anspruch 1, **dadurch gekennzeichnet**, daß die Flüssigphase einen pH-Wert von 4 bis 10 aufweist.

6. Verfahren nach Anspruch 1, **dadurch gekennzeichnet**, daß die Kontraktierung bei einem pH-Wert von 7.5 bis 11 und einer Kationenkonzentration von 0.01 bis 1 M erfolgt.

7. Verfahren nach Anspruch 2, **dadurch gekennzeichnet**, daß die Flüssigphase einen pH-Wert von 6 bis 10 aufweist.

8. Verfahren nach Anspruch 2, **dadurch gekennzeichnet**, daß die Flüssigphase multivalente Kationen enthält und daß der Puffer zur Eluierung ausreichend Chelatisierungsmittel zum Binden sämtlicher der multivalenten Kationen enthält.

9. Verfahren nach Anspruch 2, **dadurch gekennzeichnet**, daß der Schritt der Kontaktierung bei einem pH-Wert von 2 bis 7 und einer Kationenkonzentration von 10 mM bis 1M erfolgt.

10. Verfahren nach Anspruch 2, **dadurch gekennzeichnet**, daß die eluierten Nukleinsäuren doppelsträngig sind und daß der Schritt der selektiven Eluierung bei einem pH-Wert von 6 bis 10 und bei weniger als 0.1 M monovalenten Kationen und bei weniger als 0.01 M multivalenten Kationen erfolgt.

11. Verfahren nach Anspruch 2, **dadurch gekennzeichnet**, daß die eluierten Nukleinsäuren einsträngig sind und daß der Schritt der selektiven Eluierung bei einem pH-Wert von 8 bis 10 und bei weniger als 0.5 M Kationen erfolgt.

12. Verfahren nach Anspruch 3, **dadurch gekennzeichnet**, daß die Flüssigphase ausgewählt ist aus 10 mM MgCl₂, pH 7.5; und 15 mM MgCl₂, pH 8.3.

13. Verfahren nach Anspruch 3, **dadurch gekennzeichnet**, daß die Kontaktierung bei einem pH-Wert von weniger als 8.5 und bei mehr als 0.5 M Kationen erfolgt.

14. Verfahren nach Anspruch 4, **dadurch gekennzeichnet**, daß der Schritt der Kontaktierung bei einem pH-Wert von 8 bis 10 und bei weniger als 0.1 M monovalenten Kationen und weniger als 0.01 multivalenten Kationen erfolgt.

15. Verfahren zur Durchführung einer enzymatischen Reaktion zur Modifizierung von Nukleinsäuren mit den Schritten:
- Kontaktierung eines Nukleinsäuren modifizierenden Enzyms mit einem festen Substrat, welches hergestellt wurde durch das Verfahren der Anwendung einer adhäsiven Formulierung auf ein festes Substrat, wobei die adhäsive Formulierung ein nicht-proteinartiges Polymer aus monomeren Einheiten enthält, die einen aromatischen Kern aufweisen, der mit wenigstens einer Hydroxylgruppe mit einem pK-Wert von weniger als 9 substituiert ist;
- Durchführung der Kontaktierung bei einem pH-Wert von 2 bis 11 und einer Kationenkonzentration von größer als 0.1 mM, so daß das Enzym an dem festen Substrat bindet;
- Kontaktierung des festen Substrats mit gebundenem Enzym mit Nukleinsäuren unter Bedingungen, die für das Enzym geeignet sind katalytisch wirksam zu sein, wobei die Bedingungen in den Bereich des pH-Wertes von 4 bis 11 und größer als 0.1 mM Kationen fällt;
- Separierung der Nukleinsäuren von dem festen Substrat, um enzymatisch modifizierte Nukleinsäuren zu ergeben.

16. Verfahren nach Anspruch 15, **dadurch gekennzeichnet**, daß der erste Schritt der Kontraktierung zwischen pH-Wert von 4 und 10 erfolgt.

17. Vorrichtung zur Reinigung von Nukleinsäuren, welches enthält:
- ein Substrat, welches mit einer adhäsiven Formulierung enthaltend ein nicht-proteinartiges Polymer aus monomeren Einheiten aufweist, die einen aromatischen Kern aufweisen, der mit wenigstens einer Hydroxylgruppe mit einem pK-Wert von weniger als 9 substituiert ist.

18. Vorrichtung nach Anspruch 17, **dadurch gekennzeichnet**, daß der aromatische Kern eine Phenylgruppe ist.

19. Vorrichtung nach Anspruch 17 oder 18, **dadurch gekennzeichnet**, daß die Hydroxylgruppe eine para-Hydroxyl-Gruppe bezüglich der Bindungsstelle des aromatischen Kernes an dem polymeren Grundgerüst ist.

20. Vorrichtung nach einem der Ansprüche 17 bis 19, **dadurch gekennzeichnet**, daß das Substrat eine Membran ist.

21. Vorrichtung nach einem der Ansprüche 17 bis 19, **dadurch gekennzeichnet**, daß das Substrat einen Kunststoff aufweist.

22. Vorrichtung nach Anspruch 21, **dadurch gekennzeichnet**, daß der Kunststoff ausgewählt ist aus der Gruppe substituierte Polyalkane, Polyamide, Polyester, Polyäther, Polysulfone, Phenolharze, Epoxyharze und substituierte Cellulose.

23. Vorrichtung nach Anspruch 21 oder 22, **dadurch gekennzeichnet**, daß der Kunststoff in der Form von Kügelchen vorliegt.

## Revendications

1. Procédé de purification d'acides nucléiques a partir de protéines,
comprenant les étapes consistant à :
amener une composition en phase liquide comprenant une protéine et des acides nucléiques, en contact avec un substrat formé par le procédé consistant à :
• appliquer une formulation adhésive à un substrat, cette formulation adhésive comportant un polymère non protéinique d'éléments monomères comprenant une part aromatique remplacée par au moins un groupe hydroxyle ce pK inférieur à 9 ;
• cette mise en contact se faisant à un pH de 2 à 11 et à une concentration en cation supérieure à 0,1 mM, de façon que la protéine se fixe au substrat mais que les acides nucléiques ne se fixent pas au substrat ; et
• séparer la phase liquide du substrat.

2. Procédé de purification d'acides nucléiques à partir de protéines,
comprenant les étapes consistant à :
• amener une composition en phase liquide comprenant une protéine et des acides nucléiques, en contact avec un substrat formé par le procédé consistant à :
• appliquer une formulation adhésive à un substrat, cette formulation adhésive comportant un polymère non protéinique d'éléments monomères comprenant une part aromatique remplacée par au moins un groupe hydroxyle de pK inférieur à 9 ;
• cette mise en contact se faisant à un pH de 2 à 11 et à une concentration en cation supérieure à 0,1 mM, de façon que la protéine et les acides nucléiques se fixent au substrat ; et
• séparer sélectivement par élution les acides nucléiques du substrat au moyen d'un agent intermédiaire présentant un pH de 4 à 11.

3. Procédé de purification d'acides nucléiques à double brin à partir à la fois de protéines et d'acides nucléiques à une seule brin,
comprenant les étapes consistant à :
• amener une composition en phase liquide comprenant une protéine et des acides nucléiques à double brin et à simple brin, en contact avec un substrat formé par le procédé consistant à :
• appliquer une formulation adhésive à un substrat, cette formulation adhésive comportant un polymère non protéinique d'éléments monomères comprenant une part aromatique remplacée par au moins un groupe hydroxyle de pK inférieur a 9 ;
• cette mise en contact se faisant à un pH de 7 à 10 en présence de l'un ou des deux éléments constitués d'un cation monovalent à une concentration > 1 mM, et d'un cation multivalent à une concentration > 0,1 mM, de façon que la protéine et les acides nucléiques à simple brin se firent au substrat, mais que les acides nucléiques à double brin ne se fixent pas au substrat ; et
• séparer la phase liquide du substrat.

4. Procédé de séparation d'acides nucléiques à simple brin par rapport à des acides nucléiques à double brin, comprenant les étapes consistant à :
• amener une composition en phase liquide comprenant des acides nucléiques à double brin et à simple brin, en contact avec un substrat formé par le procédé consistant à :
• appliquer une formulation adhésive à un substrat, cette formulation adhésive comportant un polymère non protéinique d'éléments monomères comprenant une part aromatique remplacée par au moins un groupe hydroxyle de pK inférieur à 9 ;
• cette mise en contact se faisant à un pH de 7,0 à 10 en présence de l'un ou des deux éléments constitués d'un cation monovalent à une concentration >1 mM, et d'un cation multivalent à une concentration >0,1 mM, de façon que les acides nucléiques à simple brin se fixent au substrat mais que les acides nucléiques à double brin ne se fixent pas au substrat ; et
• séparer la phase liquide du substrat.

5. Procédé selon la revendication 1,
dans lequel
la phase liquide a un pH de 4 à 10.

6. Procédé selon la revendication 1,
dans lequel
la mise en contact se fait à un pH de 7,5 à 11 et à une concentration en cation de 0,01 à 1 M.

7. Procédé selon la revendication 2,
dans lequel
la phase liquide a un pH de 6 à 10.

8. Procédé selon la revendication 2,
dans lequel
la phase liquide comprend des cations multivalents et l'agent intermédiaire d'élution contient suffisamment d'agent chélatant pour fixer tous les cations multivalents.

9. Procédé selon la revendication 2,
dans lequel
l'étape de mise en contact se fait à un pH de 2 à 7 et à une concentration en cation de 10 mM à 1 M.

10. Procédé selon la revendication 2,
dans lequel
les acides nucléiques séparés par élution sont à double brin et l'étape d'élution sélective se fait à un pH de 6 à 10 ainsi qu'à moins de 0,1 M de cation monovalent et à moins de 0,01 M de cation multivalent.

11. Procédé selon la revendication 2,
dans lequel
les acides nucléiques séparés par élution sont à simple brin et l'étape d'élution sélective se fait à un pH de 8 à 10 ainsi qu'à moins de 0,5 M de cation.

12. Procédé selon la revendication 3,
dans lequel
la phase liquide est choisie parmi 10 mM de MgCl₂, pH 7,5 ; et 15 mM de MgCl₂, pH 8,3.

13. Procédé selon la revendication 3,
dans lequel
la mise en contact se fait à un pH inférieur d, 8,5 et à plus de 0,5 M de cation.

14. Procédé selon la revendication 4,
dans lequel
l'étape de mise en contact se fait à un pH de 8 à 10 ainsi qu'à moins de 0,1 M de cation monovalent et à moins de 0,01 M de cation multivalent.

15. Procédé pour effectuer une réaction enzymatique pour modifier des acides nucléiques,
comprenant les étapes consistant à :
amener une enzyme qui modifie les acides nucléiques, en contact avec un substrat solide réalisé par le procédé consistant à :
appliquer une formulation adhésive à un substrat solide, cette formulation adhésive comprenant un polymère non protéinique d'éléments monomères comprenant une part aromatique remplacée par au moins un groupe hydroxyle de pH inférieur à 9 ;
cette mise en contact se faisant à un pH de 2 à 11 ainsi qu'à une concentration en cation supérieure à 0,1 mM, de façon que l'enzyme se fixe au substrat solide ;
amener l'enzyme fixée au substrat solide, en contact avec les acides nucléiques dans des conditions appropriées pour que l'enzyme soit catalytiquement active, ces conditions se situant dans la plage de pH de 4 à 11 et à plus de 0,1 mM de cation ; et
séparer les acides nucléiques du substrat solide pour obtenir des acides nucléiques enzymatiquement modifiés.

16. Procédé selon la revendication 15,
dans lequel
la première étape de mise en contact se fait à un pH compris entre 4 et 10.

17. Dispositif pour purifier des acides nucléiques, comprenant :
un substrat revêtu d'une formulation adhésive comportant un polymère non protéinique d'éléments monomères comprenant une part aromatique remplacée par au moins un groupe hydroxyle de pH inférieur à 9.

18. Dispositif selon la revendication 17,
dans lequel
la part aromatique est un groupe phényle.

19. Dispositif selon la revendication 17 ou la revendication 18,
dans lequel
le groupe hydroxyle est un para-hydroxyle relatif au site de fixation de la part aromatique a l'épine dorsale du polymère.

20. Dispositif selon l'une quelconque des revendications 17 à 19,
dans lequel
le substrat est une membrane.

21. Dispositif selon l'une quelconque des revendications 17 à 19,
dans lequel
le substrat est constitué d'une matière plastique.

22. Dispositif selon la revendication 21,
dans lequel
la matière plastique est choisie parmi le polyalkène substitué, le polyamide, le polyester, le polyéther, le polysulfone, la résine phénolique, la résine époxyde, et la cellulose substituée.

23. Dispositif selon la revendication 21 ou la revendication 22,
dans lequel
la matière plastique se présente sous la forme de perles.
